# EUROPÄISCHE PATENTANMELDUNG

(11) **EP 0 747 035 A2**
(43) Veröffentlichungstag der Anmeldung: **11.12.1996**
(21) Anmeldenummer: 96109190.7
(22) Anmeldetag: 07.06.1996
(51) Int. Cl.: A61K 7/06

(54) **Mittel zur Behandlung von Kopfschuppen und zur Behandlung der Haare**

(30) Priorität: 07.06.1995 DE 19520662
(71) Anmelder: Beiersdorf Aktiengesellschaft, D-20245 Hamburg (DE)
(72) Erfinder: Sauermann, Gerhard, Dr., 24649 Wiemersdorf (DE); Riedel, Jan-Henric, Dr., 20253 Hamburg (DE); Schmidt-Lewerkühne, Hartmut, Dr., 22869 Schenefeld (DE)

(57) **Zusammenfassung**

Gegenstand der Erfindung ist die Verwendung von basischen Aminosäuren, insbesondere einer oder mehrerer Verbindungen, ausgewählt aus der Gruppe von Arginin, Ornithin, Citrullin und Lysin oder deren Salzen, Säureadditionssalzen, Estern oder Amiden, gegebenenfalls unter Zusatz von Folsäure oder deren Salzen, zur Prophylaxe und Behandlung von Kopfschuppen und zur Verbesserung der Haarfestigkeit.

## Beschreibung

Mittel zur Behandlung von Kopfschuppen und zur Behandlung der Haare.

Die Erfindung betrifft insbesondere Wirkstoffe und kosmetische und dermatologische Zubereitungen, insbesondere topische Zubereitungen, zur Prophylaxe und Behandlung von Kopfschuppen und der Haare.

Mittel zur Behandlung von Schuppen und zur Verbesserung der Haarfestigkeit sind bekannt. Ihre Wirkung ist aber nicht immer befriedigend.

Aufgabe der Erfindung ist es, Wirkstoffe und Mittel zur prophylaktischen Bekämpfung von Kopfschuppen und zur Behandlung von Kopfschuppen und zur Verbesserung der Haarfestigkeit zu schaffen, die Nachteile des Standes der Technik vermeiden.

Diese Aufgaben werden gelöst durch die Verwendung von basischen Aminosäuren, insbesondere einer oder mehreren Verbindungen, ausgewählt aus der Gruppe von Arginin, Ornithin, Citrullin und Lysin oder deren Salzen, Säureadditionssalzen, Estern, oder Amiden, gegebenenfalls unter Zusatz von Folsäure oder deren Salzen, zur Prophylaxe und Behandlung von Kopfschuppen und zur Verbesserung der Haarfestigkeit.

Gegenstand der Erfindung sind auch kosmetische und dermatologische Zubereitungen, insbesondere haarkosmetische Zubereitungen und Haarpflegeprodukte, welche die erfindungsgemäßen Wirkstoffe enthalten, und deren Verwendung zur Prophylaxe und Behandlung von Kopfschuppen und zur Verbesserung der Haarfestigkeit.

Bevorzugt werden L-Arginin, L-Ornithin, L-Citrullin und L-Lysin.

Topische Zubereitungen werden bevorzugt.

Bevorzugte Salze der Aminosäuren, insbesondere von Arginin, Ornithin, Lysin und Citrullin sind wasserlösliche Salze, z.B. Natrium-,Kalium- und Ammoniumsalze. Dies gilt auch für die Säureadditionssalze. Geeignete Säureadditionssalze werden mit anorganischen und organischen Säuren erhalten. Bevorzugt werden die Hydrochloride, Sulfate, Acetate, Caprylate oder Zitrate.

Geeignete Ester dieser Verbindungen sind z.B. solche, die mit kurzkettigen und mittelkettigen Alkoholen gebildet werden, vorzugsweise mono-Alkoholen, insbesondere aber Methanol, Ethanol oder Propanol. Bevorzugt werden die Ethylester.

Bevorzugte Amide sind kurz- und mittelkettige mono- und di-Alkylamide.

Alkyle der vorstehenden Substituenten enthalten z.B. bis zu 12, vorzugsweise bis zu 6 Kohlenstoffatome.

Besonders bevorzugt werden Arginin und Wirkstoffkombinationen und topische Zubereitungen, die Arginin und/oder dessen erfindungsgemäße Derivate enthalten.

L-Arginin und seine Derivate zeichnen sich auch durch ein besonders gutes Penetrationsvermögen aus.

Die erfindungsgemäßen basischen Aminosäuren, z.B. Arginin, Citrullin, Ornithin und Lysin und/oder ihre Derivate sind vorzugsweise in Mengen von 0,01 bis 30 Gew.-%. besonders bevorzugt 0,01 bis 10 Gew.-%, insbesondere 0,1 - 7,5 Gew.-%, jeweils bezogen auf die gesamte Zubereitung, in den erfindungsgemäßen kosmetischen und dermatologischen Zubereitungen enthalten. Die Wirkstoffe und deren Derivate können einzeln oder in Kombination eingesetzt werden. Zubereitungen enthalten jedoch vorzugsweise Arginin, besonders bevorzugt L-Arginin, in Mengen von 0,01 bis 10 Gew.-%, bezogen auf die gesamte Zubereitung.

Bevorzugt werden auch Kombinationen einer oder mehrerer der basischen Aminosäuren mit Folsäure.

Besonders geeignete Salze der Folsäure sind wasserlösliche Salze, insbesondere Natrium-, Kalium- und Ammoniumsalze.

Folsäure oder ihre Salze sind vorzugsweise in den erfindungsgemäßen Zubereitungen enthalten, bevorzugt jeweils in Mengen von 0,001 bis 5 Gew.-%, insbesondere 0,01 bis 1,5 Gew.-%, jeweils bezogen auf das Gesamtgewicht der Zubereitungen.

Die erfindungsgemäßen kosmetischen oder dermatologischen topischen Zubereitungen können auf an sich üblichen Formulierungsgrundlagen beruhen und zur Behandlung der Kopfhaut und der Haare im Sinne einer dermatologischen Behandlung oder einer Behandlung im Sinne der pflegenden Kosmetik dienen und werden in an sich bekannter Weise einmal oder mehrmals täglich an der Kopfhaut oder den Haaren angewendet.

Dermatologische und kosmetische Zubereitungen gemäß der Erfindung können in an sich bekannten Formen vorliegen. So können z.B. wäßrige, alkoholische oder wäßrig-alkoholische Lösungen, Emulsionen vom Typ Öl-in-Wasser (O/W), Emulsionen vom Typ Wasser-in-Öl (W/O), multiple Emulsionen z.B. vom Typ Wasser-in-Öl-in-Wasser (W/O/W), Gele, Hydrodispersionen, feste Stifte oder Aerosole die o.g. Wirkstoffkombinationen oder Wirkstoffe enthalten.

Bevorzugte Zubereitungen sind topische haarkosmetische Zubereitungen und zur Verwendung an der Kopfhaut geeignete und übliche Mittel.

Geeignete Haarbehandlungsmittel sind insbesondere Shampoo, Haarpflegemittel, Haarkonditioniermittel, Haarkur, Haarspülung, Haarfestiger, Haarverformungsmittel, Färbemittel, Tönungsmittel und Bleichmittel.

Die Zubereitungen gemäß der Erfindung können in verschiedener Form vorliegen, insbesondere als Lösungen, Gel, Creme, Öl, Emulsion oder jeder anderen zur Haut- und Haar-Behandlung geeigneten Form. Sie können auch als Aerosol in Gegenwart eines Treibmittels konditioniert sein.

Hierzu können, insbesondere auch für Haarbehandlungsmittel, zahlreiche kosmetisch annehmbare Bestandteile verwendet werden. Die Zubereitungen können insbesondere enthalten: anionische, kationische, nichtionische, amphotere oberflächenaktive Mittel und deren Gemische. Unter den oberflächenaktiven Mitteln sind zu nennen: Alkylbenzolsulfonate, Alkylnaphthalinsulfonate, Sulfate, Ethersulfate und Fettalkoholsulfonate, quaternäre Ammoniumsalze, Fettsäurediethanolamide, polyoxyethylierte und polyglycerinierte Säuren und Alkohole, polyoxyethylierte und polyglycerinierte Alklyphenole, sowie polyoxyethylierte Alkylsulfate. Die oberflächenaktiven Produkte liegen in den Zubereitungen gemäß der Erfindung z.B. in Anteilen zwischen 0,5 und 55 Gew.-%, vorzugsweise zwischen 4 und 40 Gew.-%, bezogen auf das Gesamtgewicht der Zubereitung vor.

Die Zubereitungen können Wasser und auch organische Lösungsmittel enthalten, um die Verbindungen zu solubilisieren, die in Wasser nicht in ausreichendem Maße löslich sind. Unter den Lösungsmitteln können z.B. genannt werden: Niedrigalkanole, wie Ethanol und Isopropanol, Glycerin, Glykole oder Glykolether, wie Butoxy-2-ethanol, Ethylenglykol, Propylenglykol, Diethylenglykolmonoethyl- und monomethylether, sowie analoge Produkte und deren Gemische. Diese Lösungsmittel liegen vorzugsweise in Anteilen von 1 bis 40 Gew.-%, insbesondere von 5 bis 30 Gew.-%, bezogen auf das Gesamtgewicht der Zubereitungen vor.

Die Zubereitungen können vorzugsweise mit Kochsalz oder mit Verbindungen aus der Gruppe Natriumalginat, Gummiarabicum, Cellulosederivate, wie Methylcellulose, Hydroxyethylcellulose, Hydroxypropylethylcellulose, Carboxymethylcellulose und verschiedene Polymere, die diese Eigenschaften besitzen, wie insbesondere Acrylsäurederivate, verdickt werden. Es ist auch möglich, mineralische Verdickungsmittel zu verwenden, wie z.B. Bentonit. Diese Verdickungsmittel liegen vorzugsweise in Anteilen zwischen 0,05 und 5 Gew.-%, bevorzugt zwischen 0,5 und 3 Gew.-%, bezogen auf das Gesamtgewicht der Zubereitungen vor.

Es ist natürlich möglich, den Zubereitungen gemäß der Erfindung jegliche andere Bestandteile zuzugeben, wie sie gewöhnlich verwendet werden, insbesondere Penetrationsagentien, Sequestrierungsagentien, filmbildenden Agentien, Puffer und Parfüme.

Schließlich können in den Zubereitungen noch weitere übliche kosmetische Zusätze, beispielsweise Antioxidantien wie Ascorbiensäure oder Natriumsulfit, Alkalisierungsmittel wie Alkalihydroxide, Ammonium- bzw. Alkalicarbonat und Ammonium- bzw. Alkalihydrogencarbonat, organische Säuren wie z.B. Essigsäure, Milchsäure und Zitronensäure, Lösungsmittel, Parfüm, Quellmittel, Netzmittel, Emulgatoren, Pflegestoffe und andere vorhanden sein.

Je nach Zusammensetzung können die erfindungsgemäßen Zubereitungen schwach sauer, neutral oder alkalisch reagieren.

Die erfindungsgemäßen topischen Zubereitungen, die insbesondere auch zur Behandlung der Kopfhaut dienen, können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylelher, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Kosmetische und dermatologische Zubereitungen zur Behandlung und Pflege der Haut können als Gele vorliegen, die neben den Wirkstoffen und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Erfindungsgemäße Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen, metastabile Systeme dar und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Feste Stifte gemäß der Erfindung können z.B. natürliche oder synthetische Wachse, Fettalkohole oder Fettsäureester enthalten.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Die erfindungsgemäßen, z.B. topischen Zubereitungen, können die üblichen Hilfsstoffe, Emulgatoren und Konservierungsmittel enthalten.

Die erfindungsgemäßen Wirkstoffe, Wirkstoffkombinationen und die Zubereitungen, die sie enthalten, besitzen eine ausgeprägte Antischuppenwirkung. Sie sind zur Behandlung der Kopfschuppen und zur prophylaktischen Behandlung der Schuppenbildung der Kopfhaut und deren Schuppigkeit geeignet. Weiterhin erhöhen sie die Festigkeit der Haare. Sie dienen der Vorbeugung und Behandlung von gesplissenem Haar (Spliss) oder gebrochenem Haar oder in anderer Weise beschädigtem Haar, insbesondere dem Kopfhaar.

Gegenstand der Erfindung ist auch das Verfahren zur Herstellung der erfindungsgemäßen, z. B. topischen Zubereitungen, das dadurch gekennzeichnet ist, daß man in an sich bekannter Weise die Wirkstoffe in kosmetische oder dermatologische Formulierungen einarbeitet.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

### Beispiel 1

| Shampoo (CTFA) | Gew.-% |
|---|---|
| Natriumlaurylethersulfat (2 EO, 20%) | 40 |
| Lauryl dimethylamino acetic acid betaine (30%) | 4 |
| Cocamide DEA, Coconut fatty acid diethanolamine | 1.5 |
| Oleth-3 phosphate, Oleyl triethoxy phosphate, (Briphos O3D, Albright & Wilson) | 1 |
| PEG-15 cocopolyamine, Polyglycol-polyamine condensation resin (Polyquart H, Henkel, 50%) | 2 |
| L-Argininhydrochlorid | 5 |
| Wasser, VES (vollentsalzt) | ad 100 |

### Beispiel 1 a

Es wird wie in Beispiel 1 verfahren, und es werden 0,5 Gew.-% Folsäure zugegeben.

### Beispiel 2

| Hydrodispersion | Gew.-% |
|---|---|
| L-Ornithinhydrochlorid | 1,0 |
| Phenyltrimethicon | 1,0 |
| Carbomer (Carbopol 981) | 1,0 |
| Hydroxypropylmethylcellulose | 0,2 |
| Butylenglycol | 3,0 |
| Tromethamin | q.s. |
| NaOH-Lösung (15-Gew.-%ig) | q.s. |
| Ethanol | 5,0 |
| Parfum, Konservierungsmittel | q.s. |
| Wasser, VES | ad 100,0 |

### Beispiel 2 a

An Stelle von L-Ornithinhydrochlorid wird die gleiche Gewichtsmenge L-Argininhydrochlorid zugegeben.

### Beispiel 3

| Lotion W/O | Gew.-% |
|---|---|
| L-Argininhydrochlorid | 0,5 |
| Folsäure | 0,1 |
| Cyclomethicon | 3,0 |
| PEG-1-Glycerin Sorbitan Oleostearat | 1,7 |
| PEG-7 Hydriertes Rizinusöl | 6,3 |
| Mineralöl (DAB 9) | 13,9 |
| Caprylic/capric Triglyceride | 13,0 |
| Magnesiumsulfat | 0,7 |
| Parfum, Konservierungsmittel | q.s. |
| Wasser, VES | ad 100 |

### Beispiel 4

| Haarwasser (Tonikum) | Gew.-% |
|---|---|
| L-Arginin | 0,5 |
| Ethanol | 50 |
| Wasser | ad 100 |

### Beispiel 4 a

Es wird wie in Beispiel 4 angegeben verfahren, und es werden zusätzlich 0,5 Gew.-% Folsäure zugegeben.

### Beispiel 5

| W/O-Creme | Gew.-% |
|---|---|
| L-Argininhydrochlorid | 2,5 |
| L-Ornithinhydrochlorid | 2,5 |
| Folsäure | 0,1 |
| PEG-22-Dodecyl Glycol Copolymer | 3,0 |
| Cetyl Dimethicon Copolyol | 2,0 |
| Cyclomethicon | 4,0 |
| Mineralöl (DAB 9) | 4,0 |
| Caprylic/capric Triglyceride | 4,0 |
| Glycerin | 4,00 |
| Parfum, Konservierungsmittel | q.s. |
| Wasser, VES | ad 100,00 |

### Beispiel 6

In den vorstehenden Beispielen können auch jeweils L-Citrullin oder L-Lysin in der Form ihrer Hydrochloride in den gleichen Gewichtsmengen an Stelle von Arginin oder Ornithin verwendet werden.

Die vorstehenden Zubereitungen können an der Kopfhaut und/oder den Haaren angewendet werden.

## Patentansprüche

1. Verwendung von basischen Aminosäuren, insbesondere einer oder mehrerer Verbindungen, ausgewählt aus der Gruppe von Arginin, Ornithin, Citrullin und Lysin oder deren Salzen, Säureadditionssalzen, Estern oder Amiden, gegebenenfalls unter Zusatz von Folsäure oder deren Salzen, zur Prophylaxe und Behandlung von Kopfschuppen und zur Verbesserung der Haarfestigkeit.

2. Kosmetische und dermatologische Zubereitungen, insbesondere haarkosmetische Zubereitungen und Haarpflegeprodukte, welche die erfindungsgemäßen Wirkstoffe gemäß Anspruch 1 enthalten, und deren Verwendung zur Prophylaxe und Behandlung von Kopfschuppen und zur Verbesserung der Haarfestigkeit.

3. Wirkstoffe und Zubereitungen gemäß Anspruch 1 oder 2, dadurch gekennzeichnet, daß die Wirkstoffe L-Arginin, L-Ornithin, L-Citrullin und/oder L-Lysin und/oder deren Derivate bzw. Salze sind.

4. Wirkstoffe und Zubereitungen gemäß den vorstehenden Ansprüchen, gekennzeichnet durch einen Gehalt an oder Zusatz von Folsäure und/oder deren Salzen.
